# NEUE EUROPÄISCHE PATENTSCHRIFT

(11) **EP 0 859 006 B2**
(45) Veröffentlichungstag und Bekanntmachung des Hinweises auf die Entscheidung über den Einspruch: **13.09.2006**
(45) Hinweis auf die Patenterteilung: 23.04.2003
(21) Anmeldenummer: 98100935.0
(22) Anmeldetag: 21.01.1998
(51) Int. Cl.: C07H 15/04

(54) **Verfahren zur Herstellung alpha-D-Glucopyranosyl-(1-6)-D-sorbit- oder alpha-D-Glucopyranosyl-(1-6)-D-mannit-reicher Gemische aus hydrierter Isomaltulose**
Process for the preparation of mixtures enriched in alpha-D-glucopyranosyl-(1-6)-D-sorbitol or alpha-D-glucopyranosyl-(1-6)-D-mannitol from hydrogenated isomaltulose.
Procédé de préparation de compositions contenant alpha-D-glucopyranosyl-(1-6)-D-sorbitol ou alpha-D-glucopyranosyl-(1-6)-D-mannitol à partir d'isomaltulose hydrogénée

(30) Priorität: 14.02.1997 DE 19705664
(43) Veröffentlichungstag der Anmeldung: 19.08.1998
(73) Patentinhaber: Südzucker Aktiengesellschaft Mannheim/Ochsenfurt, 68165 Mannheim (DE)
(72) Erfinder: Degelmann, Hanspeter, 67549 Worms (DE); Gander, Michael, 67550 Worms (DE); Kowalczyk, Jörg, Dr., 67248 Bockenheim (DE); Kunz, Markwart, Dr., 67550 Worms (DE); Munir, Mohammad, Dr., 67271 Kindenheim (DE); Wach, Wolfgang, Dr., 67549 Worms (DE); Schüttenhelm, Matthias, Dr., 67547 Worms (DE)
(74) Vertreter: Schrell, Andreas

(56) Entgegenhaltungen:
- DE-A- 2 520 173
- DE-B- 2 217 628
- Alimenta, Band 19, S. 5-16 (1980)
- USSR Patent 559926 in englischer Uebersetzung

## Beschreibung

Die vorliegende Erfindung betrifft ein Verfahren zur Herstellung 6-O-α-D-Glucopyranosyl-D-sorbit-(imfolgenden 1,6-GPS genannt) und/oder 1-O-α-D-Glucopyranosyl-D-mannit- (im folgenden 1,1-GPM genannt) angereicherter Gemische aus hydrierter Isomaltulose oder aus Gemischen, die hydrierte Isomaltulose enthalten.

Hydrierte Isomaltulose, ein nahezu äquimolares Gemisch aus 1,6-GPS und 1,1-GPM, ist ein kommerziell erhältlicher Zuckeraustauschstoff, der aufgrund seiner Akariogenität, seines geringen Brennwerts und seiner Diabetikereignung vorteilhaft ist. 1,6-GPS sowie 1,1-GPM in reiner Form oder Gemische, die 1,6-GPS oder 1,1-GPM in größerer Menge enthalten, sind für eine Vielzahl von Anwendungen beispielsweise im Lebensmittelbereich besser geeignet, als die derzeitig bekannten Produkte, die in etwa äquimolare Verhältnisse von 1,1-GPM/1,6-GPS aufweisen. Die Druckschrift DE 25 20 173 A1 betrifft ein Verfahren zur Herstellung von 1,1-GPM aus Isomaltulose sowie dessen Verwendung als Zuckeraustauschstoff. Ein Herstellungsverfahren für 1,6-GPS-angereicherte oder 1,1-GPM-angereicherte Gemische sowie 1,6-GPS und 1,1-GPM in reiner Form, das eine einfache und effiziente Herstellung aus hydrierter Isomaltulose gestattet, ist derzeit aber nicht bekannt.

Der Erfindung liegt daher das technische Problem zugrunde, ein Herstellungsverfahren für 1,1-GPM und 1,6-GPS sowie diese Stoffe in größerer Menge enthaltende Gemische bereitzustellen, das einfach durchzuführen ist und in hoher Ausbeute zu möglichst reinen Endprodukten führt.

Die vorliegende Erfindung löst das technische Problem durch die Bereitstellung eines Verfahrens zur Herstellung 1,1-GPM- und 1,6-GPS-angereicherter Gemische beziehungsweise von reinem 1,6-GPS und reinem 1,1-GPM gemäß Hauptanspruch.

Die Erfindung sieht demgemäß ein Verfahren zur Herstellung 1,1-GPM- und 1,6-GPS-angereicherter Phasen vor, wobei hydrierte Isomaltulose oder ein hydrierte Isomaltulose-haltiges Gemisch in Wasser bei erhöhter Temperatur gelöst, mit der erhaltenen Lösung eine Kühlungskristallisation über eine gegebene, zum Erhalt der gewünschten Phasenzusammensetzung ausreichende Temperaturspanne durchgeführt und anschließend die erhaltene 1,1-GPM-reiche Phase von der erhaltenen 1,6-GPS-reichen Phase abgetrennt wird. Die Kühlungskristallisation kann entweder diskontinuierlich oder kontinuierlich durchgeführt werden.

Die Erfindung sieht vor, daß die Kühlungskristallisation der erhaltenen Lösung in einem Temperaturbereich von 90 °C bis 65 °C mit einer Kühlrate von 5 bis 15 K/h und in einem Temperaturbereich von 65 °C bis 37 °C, mit einer Kühlrate von 0.4 bis 3 K/h durchgeführt wird.

Im Zusammenhang mit der vorliegenden Erfindung wird unter hydrierter Isomaltulose ein äquimolares oder nahezu äquimolares Gemisch aus 1,1-GPM und 1,6-GPS verstanden. Unter einem hydrierte Isomaltulose-haltigen Gemisch wird ein Gemisch aus 1,1-GPM, 1,6-GPS sowie einem odermehreren der folgenden beispielhaft genannten Stoffe verstanden: Mannit, Sorbit, Saccharose, 1,1-GPS (1-O-α-D-Glucopyranosyl-D-sorbit), Isomaltose, hydrierte oder nicht hydrierte Oligosaccharide, Isomelezitose oder andere Stoffe. Für das vorliegende Verfahren geeignete Rohstoffe sind beispielsweise die in der EP 0 625 578 B1 beschriebenen Süßungsmittel.

Im Zusammenhang mit der vorliegenden Erfindung wird unter einer erhöhten Temperatur eine Temperatur verstanden, bei der eine vollständige oder weitgehend vollständige Lösung der aufzulösenden hydrierten Isomaltulose oder des aufzulösenden hydrierte Ismaltulose-haltigen Gemisches in der gewünschten zur Kühlungskristallisation eingesetzten Ausgangskonzentration, nämlich 70 bis 90 Gew.-% a.TS (auf Trokkensubstanz), in Wasser möglich ist.

Die Erfindung sieht vorteilhafterweise vor, daß in einem Temperaturbereich von 90 °C bis 65 °C eine Kühlrate von 5 bis 15 K/h und in einem Temperaturbereich von 65 °C bis 37 °C, eine Kühlrate von 0,4 bis 3 K/h, einzuhalten ist. Diese Angaben sind jedoch nicht so zu verstehen, daß die Kühlungskristallisation den vollen genannten Temperaturbereich von 90 °C bis 37 °C ausnutzt oder nicht darüber hinausgeht. Diese Angaben sind vielmehr so zu verstehen, daß, soweit die Kühlungskristallisation in dem jeweils genannten Temperaturbereich stattfindet, eine derartige Kühlrate einzuhalten ist. Die Temperaturwerte, bei derdie Kühlungskristallisation beginnt und bei der sie beendet wird, das heißt die gegebene Temperaturspanne, hängen vielmehr unter anderem von der eingesetzten Konzentration des Rohstoffes, der Zusammensetzung des Rohstoffes und dem gewünschten Reinheitsgrad des Produktes ab und können durch einfache Versuche ermittelt werden. Die Kühlungskristallisation wird bevorzugt über eine Temperaturspanne durchgeführt, die ausreicht, Kristalle in für eine effiziente Abtrennung geeigneter Größe zu erhalten und/oder die ausreicht, eine ausreichende Anreicherung von 1,1-GPM bzw. 1,6-GPS in den erhaltenen Phasen zu gewährleisten, beispielsweise von über 70 Gew.-% a.TS, bevorzugt über 80 Gew.-% a.TS und besonders bevorzugt über 85 Gew.-% a.TS an 1,1-GPM bzw. 1,6-GPS. Gleichwohl sieht die Erfindung in besonders bevorzugter Weise vor, die Kühlungskristallisation bei einer Temperatur von 90 °C bis 80 °C, bevorzugt 85 °C, zu beginnen und bis zu einer Temperatur von 40 °C bis 37°C durchzuführen.

Die Erfindung stellt also ein besonders einfaches und effizientes Verfahren zur Herstellung von reinem 1,1-GPM und reinem 1,6-GPS oder diese Stoffe in angereicherter, d. h. über 50 Gew.-% a.TS enthaltend, Menge aufweisende Gemische zur Verfügung, das sich eine unterschiedlich gute Löslichkeit von 1,1-GPM und 1,6-GPS zu Nutze macht. In besonders bevorzugter und vorteilhafter Weise bestehen die aus der erfindungsgemäß vorgesehenen Kühlungskristallisation erhaltenen Kristallisate aus besonders grobkörnigen Kristallen, die sich für eine anschließend erfolgende Abtrennung besonders gut eignen. Insbesondere aufgrund der erfindungsgemäß einzusetzenden Kühlrate von 5 bis 15 K/h in einem Temperaturbereich von 90 °C bis 65 °C und von 0,4 bis 3 K/h bei 65 °C bis 37 °C wird eine besonders gleichmäßige Kristallgrößenverteilung im erhaltenen 1,1-GPM-angereicherten Produkt erhalten, so daß dieses gut abzentrifugiert werden kann. Bei Nichteinhaltung der erfindungsgemäßen Verfahrensweise, insbesondere der Kühlraten, ist eine Abtrennung der 1,1-GPM-reichen Kristalle durch Zentrifugation nicht realisierbar, insbesondere weil die erhaltene Kristallgrößenverteilung zu heterogen ist und die erhaltenen Kristalle nicht die erforderliche mittlere Kristallgröße aufweisen (erhaltene Kristalle sind zu klein), die für eine effiziente Zentrifugation ausreicht. Die schnelle und gute Abtrennung der nach der Kühlungskristallisation erhaltenen Mutterlauge vom Kristallisat durch die erfindungsgemäß bevorzugt vorgesehene Zentrifugation macht die Kühlungskristallisation gemäß der vorliegenden Erfindung für eine effiziente Herstellung der genannten Produkte besonders geeignet.

Die Erfindung geht davon aus, daß sich die Löslichkeiten von 1,1-GPM und 1,6-GPS in Wasser unterscheiden und sich die Löslichkeitsgleichgewichte der beiden Komponenten bereits nach kurzer Zeit, vorzugsweise bereits nach einer halben Stunde, eingestellt haben. Da die Löslichkeitsgleichgewichte temperaturabhängig sind, ist es erfindungsgemäß möglich, den Anreicherungsgrad der beiden Komponenten gezielt einzustellen. Erfindungsgemäß kann also nach Einstellung des Löslichkeitsgleichgewichtes durch eine gezielte Temperaturführung eine gezielte Anreicherung von 1,1-GPM und 1,6-GPS in Produkten erreicht werden.

Die Erfindung sieht vor, daß beispielsweise zunächst eine Suspension aus hydrierter Isomaltulose oder eines hydrierte Isomaltulose-haltigen Gemisches in Wasser hergestellt wird, diese Suspension durch Erhöhung der Temperatur aufgelöst und mit der erhaltenen Lösung eine Kühlungskristallisation durchgeführt wird.

Die Erfindung sieht auch vor, daß als Rohstoff eine durch Hydrierung bereits mit den gewünschten 1,6-GPS- oder 1,1-GPM-Komponenten angereicherte Lösung verwendet wird, die aufkonzentriert, zum Beispiel durch Eindampfung, und anschließend der Kühlungskristallisation zugeführt wird.

Die Erfindung sieht ferner vorteilhafterweise vor, daß der beispielsweise in kristalliner Form vorliegende Ausgangsstoff hydrierte Isomaltulose oder eines der in der EP 0 625 578 B1 beschriebenen Süßungsmittel in Wasser bei einer Temperatur von 80 °C bis 90 °C, insbesondere 85 °C, gelöst wird. Die Konzentration der erhaltenen Lösung liegt vorteilhafterweise bei 70 bis 90 Gew.-% a.TS, besonders bevorzugt bei 75 - 85 Gew.-% a.TS.

Die Erfindung sieht in einer besonders bevorzugten Ausführungsform vor, die Kühlungskristallisation im Temperaturbereich von 90 °C bis 65 °C bei einer Kühlrate von 8 bis 12 K/h durchzuführen. In einer weiteren bevorzugten Ausführungsform sieht die Erfindung vor, die Kühlungskristallisation im Temperaturbereich von 65 °C bis 37 °C, mit einer Kühlrate von 0,8 bis 1,5 K/h durchzuführen.

Die Erfindung sieht ferner vor, daß, nachdem eine durch Zugabe von beispielsweise kristalliner hydrierter Isomaltulose in Wasser gebildete Suspension durch Temperierung auf beispielsweise 85 °C vollständig gelöst wurde, die sich anschließende Kühlungskristallisation vorzugsweise unter Einschluß eines Impfschrittes durchgeführt wird. Der Impfschritt ist jedoch nicht unbedingt erforderlich. In vorteilhafter Weise sieht die Erfindung vor, pulverisierte hydrierte Isomaltulose in kristalliner Form oder als Suspension einzusetzen. Besonders bevorzugt ist eine Suspension in Wasser oder einem organischen Lösungsmittel, zum Beispiel Isopropanol, gegebenenfalls unter Zuhilfenahme eines für Lösungsmittelzwecke geeignetes Dispergierhilfsmittels. Außerdem kann anstatt von hydrierter Isomaltulose reines 1,1-GPM oder 1,6-GPS je nach gewünschtem Produkt als Suspensionsrohmaterial eingesetzt werden. Nach Einbringen der Impfsuspension in die konzentrierte Lösung aus beispielsweise hydrierter Isomaltulose lösen sich die leichter löslichen 1,6-GPS-Kristalle vollständig, während die schwer löslichen 1,1-GPM-Kristalle als Keime verbleiben. Erfindungsgemäß konnte gefunden werden, daß die anschließend erhaltenen 1,1-GPM-reichen Kristalle besonders groß sind, so daß eine gegebenenfalls anschließende Zentrifugation des erhaltenen Kristallbreis zu einer guten Feststoffabtrennung führt. Eine leichte Trübung des Zentrifugats kann durch eine optional nachgeschaltete Druckfiltration beseitigt werden.

Die Erfindung sieht also auch vor, daß im Anschluß an die Kühlungskristallisation eine Abtrennung der 1,1-GPM-angereicherten Phase, also des Kristallisats, beispielsweise mittels Zentrifugation, Filtration oder Sedimentation, von der 1,6-GPS-angereicherten wäßrigen Phase durchgeführt wird. Die erfindungsgemäß vorgesehene bevorzugte Zentrifugation führt zu einer Abtrennung einer Mutterlauge von dem Kristallisat. Das Kristallisat ist 1,1-GPMreich, während die Mutterlauge 1,6-GPS-reich ist. Das 1,1-GPM-reiche Kristallisat enthält 1,1-GPM bereits in einer Reinheit von über 75 Gew.-% a.TS und kann in weiteren Aufreinigungs- und Konzentrierungsschritten zu reinem kristallinen 1,1-GPM mit einer Reinheit von über 99 Gew.-% a.TS weiterverarbeitet werden. Die 1,6-GPS-reiche Mutterlauge enthält 1,6-GPS bereits zu einem Anteil von über 80 Gew.-% a.TS und kann in weiteren Aufreinigungs- und Konzentrierungsschritten zu reinem kristallinen 1,6-GPS mit einer Reinheit von über 99 Gew.-% a.TS weiterverarbeitet werden.

Die Gewinnung von reinem 1,1-GPM aus dem 1,1-GPM-reichen Kristallisat kann erfindungsgemäß erfolgen, indem zunächst das Kristallisat in Wasser, beispielsweise bei einer Temperatur von 55 °C bis 65 °C, bevorzugt 60 °C, aufgelöst, gegebenenfalls filtriert und anschließend im Vakuum, beispielsweise bei 85 °C bis 90 °C, bevorzugt 88 °C, aufkonzentriert wird. Die aufkonzentrierte Lösung kann erfindungsgemäß einer ersten Kühlungskristallisation unterzogen werden, wobei in Temperaturbereichen von 90 °C, bevorzugt 88 °C, bis 75 °C eine Abkühlrate von 9 K/h und in Temperaturbereichen von 65°C bis 37 °C eine Abkühlrate von 1 K/h eingehalten wird. Im Anschluß an die erste Kühlungskristallisation wird eine Zentrifugation durchgeführt, wobei eine 1,6-GPS-reiche Mutterlauge abgetrennt wird. Das resultierende Kristallisat weist bereits einen 1,1-GPM-Gehalt von 95 Gew.-% a.TS auf und wird, vorzugsweise bei 90 °C bis 100 °C, bevorzugt bei 95 °C, in Wasser gelöst. In bevorzugter Weise schließt sich eine zweite Kühlungskristallisation an, wobei die Abkühlrate in einem Temperaturbereich von 95 °C bis 65 °C bei 3 K/h und in einem Temperaturbereich von 75 °C bis 40 °C, vorzugsweise von 65 °C bis 40 °C, mit einer Abkühlrate von 1 K/h durchgeführt wird. Eine sich gegebenenfalls anschließende Zentrifugation führt zu einer 1,1-GPM-reichen Mutterlauge sowie zu einem 1,1-GPM-reichen Kristallisat mit einer Reinheit von 99 Gew.-% a.TS an 1,1-GPM.

Die Gewinnung von reinem 1,6-GPS aus der Mutterlauge, die aus der zur Abtrennung der 1,6-GPS-reichen Phase von der 1,1-GPM-reichen Phase durchgeführten Kühlungskristallisation resultiert, kann erfindungsgemäß erfolgen, indem die 1,6-GPS-reiche Mutterlauge beispielsweise bei 50 °C bis 60 °C, vorzugsweise 55 °C, aufkonzentriert und anschließend kühlungskristallisiert wird. Dabei werden in einem Temperaturbereich von 60 °C, bevorzugt 55 °C, bis 40 °C Kühlraten von 0,3 K/h bevorzugt. Die Aufkonzentrierung und die Kühlungskristallisation werden gegebenenfalls unter gleichen Bedingungen wiederholt, bis eine ausreichende Kristallmenge und ein ausreichender Kristalldurchmesser erreicht wird. Die sich daran anschließende Zentrifugation führt zu einer 1,6-GPS-reichen Mutterlauge und einem 1,6-GPS-reichen Kristallisat mit einer Reinheit von 99 Gew.-% a.TS an 1,6-GPS.

Selbstverständlich gilt auch bei den vorgenannten, im Verlauf der Gewinnung von reinem 1,1-GPM und reinem 1,6-GPS eingesetzten Kühlungskristallisationen, daß die angegebenen Temperaturbereiche für die Kühlraten nicht zwangsläufig bedeuten, daß die jeweilige Kristallisation über den gesamten angegebenen Temperaturbereich durchgeführt wird oder nicht darüber hinaus geht. Die Anfangs- und Endwerte der bei den jeweiligen Kühlungskristallisation eingesetzten Temperaturen, d. h. die Temperaturspannen, richten sich vielmehr nach Art und Konzentration der Edukte und den gewünschten Produktzusammensetzungen (Kristallisationsgrad, Kristalldurchmesser, Reinheit etc.). Die Temperaturspannen liegen bei der Gewinnung von 1,1-GPM bevorzugt bei einer ersten Kristallisation bei 88 °C bis 37 °C und bei einer zweiten Kristallisation bei 95 °C bis 40 °C und bei der Gewinnung von 1,6-GPS bei 55 °C bis 40 °C.

Erfindungsgemäß kann ferner vorgesehen sein, die erhaltenen 1,1-GPM und 1,6-GPS-reichen Produkte beziehungsweise 1,1-GPM und 1,6-GPS in reiner Form zur Verbesserung ihrer Lagerfähigkeit zu trocknen. Die Trocknung von 1,6-GPS erfolgt erfindungsgemäß, indem das feuchte 1,6-GPS bei 40 °C bis 50 °C, insbesondere 45 °C, über beispielsweise 5 Stunden getrocknet wird. Die Trocknung von 1,1-GPM erfolgt erfindungsgemäß in bevorzugter Weise, indem ausgehend von einer Anfangstrocknungstemperatur von beispielsweise 35 °C mit einer Heizrate von 1 K/h sechs Stunden lang getrocknet wird.

Die an 1,1-GPM beziehungsweise an 1,6-GPS angereicherten Lösungen oder das reine 1,1-GPM beziehungsweise 1,6-GPS können in einer weiteren Ausführungsform der vorliegenden Erfindung bis auf 60 bis 90 % Trockensubstanzgehalt eingedickt werden, wobei anschließend das trockene gebrauchsfähige Produkt durch eine Verdampfungskristallisation mit und ohne Einsatz von Vakuum isoliert werden kann. Als vorteilhaft hat sich bei dieser Methode gezeigt, daß eine Vorlage von bereits getrocknetem Endprodukt die Durchführung vereinfacht.

Weitere vorteilhafte Ausgestaltungen der Erfindung sind den Unteransprüchen zu entnehmen.

Die Erfindung wird anhand von Ausführungsbeispielen und dazugehöriger Zeichnungen näher erläutert.

Die Figuren zeigen:
- Figur 1: schematisch den Verfahrensverlauf zur Herstellung 1,1-GPM- und 1,6-GPS-angerei-cherter Phasen,
- Figur 2: schematisch den Verfahrensverlauf zur Herstellung von reinem 1,1-GPM,
- Figur 3: schematisch den Verfahrensverlauf zur Herstellung von reinem 1,6-GPS und
- Figur 4: ein Löslichkeitsdiagramm von hydrierter Isomaltulose, Isomaltulose, 1,1-GPM und 1,6-GPS.

### Beispiel 1:

### Auftrennung von hydrierter Isomaltulose In 1,1-GPM - und 1,6-GPS-reiche Phasen mittels Kühlungskristallisation

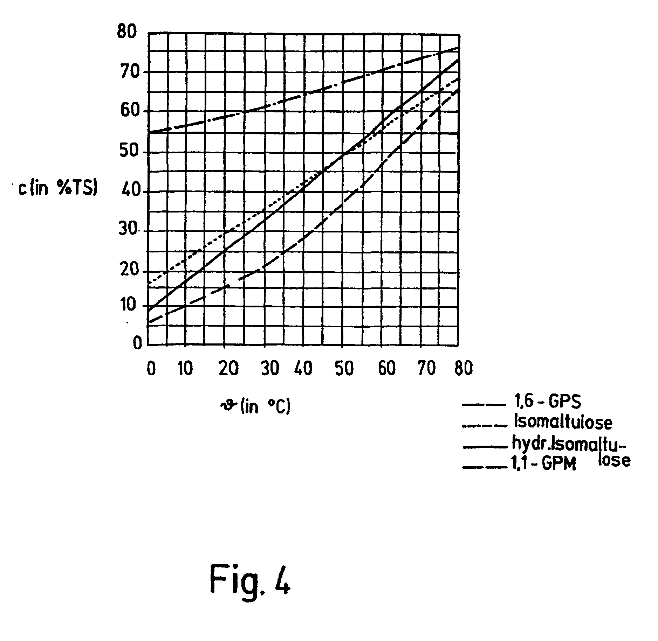

Die beiden Bestandteile von hydrierter Isomaltulose, das heißt 1,1-GPM und 1,6-GPS, lösen sich ungleich gut in wässrigen Lösungen (Figur 4). In Suspension bildet jede Komponente ihr eigenes Löslichkeitsgleichgewicht aus. Es kommt zu einer Anreicherung der leichter kristallisierenden Komponente 1,1-GPM im Feststoffanteil der Suspension, da die schwerer kristallisierende Komponente 1,6-GPS bevorzugt in Lösung geht. Die Ausbildung der Löslichkeitsgleichgewichte der beiden Komponenten dauert etwa 0,5 Stunden bei homogener Verteilung. Die sich ausbildenden Gleichgewichte sind bei gleicher Konzentration der Suspension an hydrierter Isomaltulose in Wasser temperaturabhängig. Bei gleicher Temperatur nimmt der 1,6-GPS-Anteil in der Lösung mit steigendem TS-Anteil der Suspension zu. Das in Figur 4 gezeigt Löslichkeitsdiagramm von 1,1-GPM, 1,6-GPS und hydrierter Isomaltulose zeigt eine stetige Zunahme der Löslichkeit der einzelnen Komponenten bei steigenden Temperaturen. Diese Beobachtung macht sich die vorliegende Erfindung zunutze.

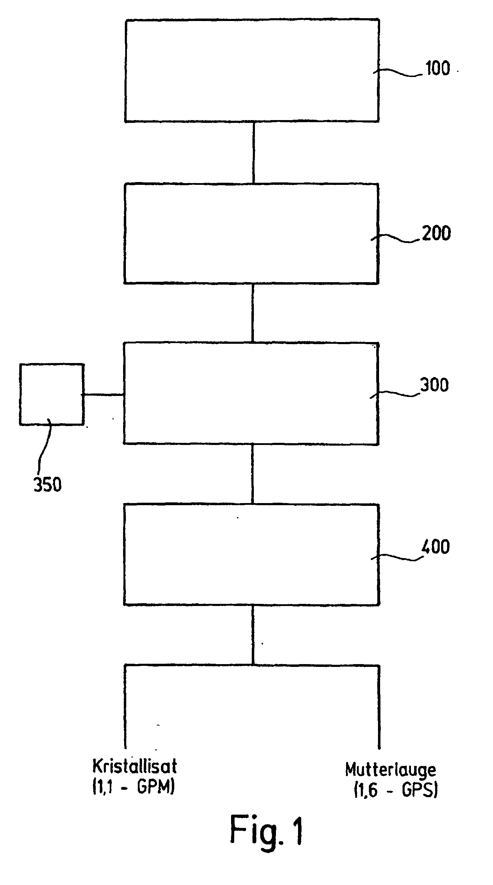

1.000 kg hydrierte Isomaltulose werden in 265 kg Wasser gelöst (Verfahrensschritt 100, Figur 1). Die dabei entstehende Suspension wird zwei Stunden bei 85 °C erhitzt (Verfahrensschritt 200, Figur 1), so daß die hydrierte Isomaltulose vollständig in Lösung geht. Es ergibt sich eine Eduktkonzentration von 77 Gew.-% a.TS. Die Lösung wird anschließend beginnend bei einer Temperatur von 85 °C und endend bei einer Temperatur von 37 °C kühlungskristallisiert, und zwar in einem Temperaturbereich von 85 °C bis 65 °C mit einer Kühlungsrate von 10,0 K/h. In dem Temperaturbereich von 65 °C bis 37 °C wurde eine Kühlungsrate von 1,2 K/h eingestellt (Verfahrensschritt 300). Bei einer Temperatur von 62 °C wurde pulverisierte hydrierte Isomaltulose in Isopropanolsuspension zugegeben (Verfahrensschritt 350, Figur 1). Im anschließenden Zentrifugationsschritt 400 werden große 1,1-GPM-reiche Kristalle erhalten, die eine gute Abtrennung von der 1,6-GPS-reichen Mutterlauge ermöglichen. Die Zentrifugation wird bei 1.800 U/min über bis zu 0,5 Stunden durchgeführt. Dabei kann vorgesehen sein, Waschwasser, insbesondere 16 kg (1 kg pro Füllung) zuzugeben. Der bei der Zentrifugation anfallende überstand kann 1 bis 2 Stunden bei 6 bar filtriert werden, wobei zusätzlich Kristallisat abgetrennt wird. Der 1,1-GPM-Anteil im Kristallisat (561 kg Kristallisat) beträgt 77,4 Gew.-% a.TS, während der 1,6-GPS im Kristallisat 22,1 Gew.-% a.TS beträgt. Der 1,1-GPM-Anteil in der Mutterlauge beträgt 13,8 Gew.-% a.TS, während der 1,6-GPS-Anteil in der Mutterlauge (560 kg Mutterlauge) 84,0 Gew.-% a.TS beträgt.

### Beispiel 2:

### Herstellung von reinem 1,1-GPM

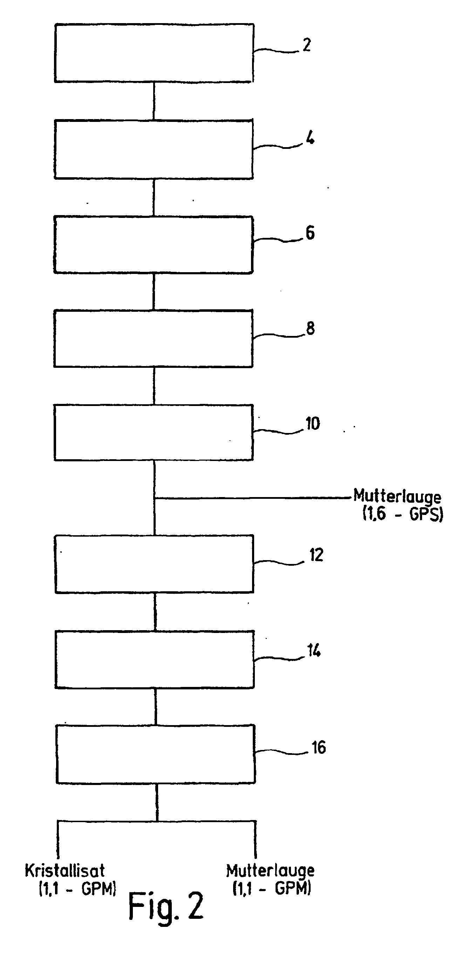

Ausgehend von dem in Beispiel 1 erhaltenen 1,1-GPM-reichen Kristallisat mit einer Reinheit von 77 Gew.-% a.TS wurden 561 kg dieses Kristallisats in 442 kg Wasser aufgelöst (Verfahrensschritt 2, Figur 2). Dabei wurde eine Temperatur von 60 °C eingestellt. Die Lösung wurde mittels einer Rahmenfilterpresse filtriert, um Verunreinigungen (zum Beispiel Dichtungsabrieb von Apparaturen abzutrennen (Verfahrensschritt 4, Figur 2)). Anschließend wurde die Lösung bei 88 °C auf 81 Gew.-% a.TS im Vakuum (0,02 bar) aufkonzentriert (Verfahrensschritt 6, Figur 2). Im Anschluß daran wird eine erste Kühlungskristallisation von 88 °C bis 37 °C (Verfahrensschritt 8) durchgeführt, wobei in einem Temperaturbereich von 88 °C bis 75 °C eine Abkühlrate von 9 K/h und in einem Temperaturbereich von 75 °C bis 37 °C eine Abkühlrate von 1 K/h eingehalten wurde. Diese erste Umkristallisation ergibt nach Durchführung einer Zentrifugation über 2 Stunden bei 1.800 U/min (Verfahrensschritt 10) eine Aufreinigung des 1,1-GPM von 77 Gew.-% a.TS auf 95 Gew.-% a.T. Die bei der Zentrifugation entstehende Mutterlauge ist 1,6-GPS-reich. Das 1,1-GPM-reiche Kristallisat (200 kg von 334 kg erhaltenem Kristallisat mit 95 Gew.-% a.TS) wird mit wenig Wasser bei 95 °C gelöst (Verfahrensschritt 12) und erneut einer Kühlungskristallisation, in diesem Fall von 95 °C bis 40 °C (Verfahrensschritt 14, Figur 2), unterworfen. Diese zweite Umkristallisation erfolgt in einem Temperaturbereich von 95 °C bis 65 °C mit einer Abkühlrate von 3 K/h und in einem Temperaturbereich von 65 °C bis 40 °C mit einer Abkühlrate von 1 K/h. Nach Zentrifugation bei 2.900 U/min über zwei Stunden wurden 158,0 kg eines Kristallisats mit 99 Gew.-% a.TS 1,1-GPM sowie eine 1,1-GPM-reiche Mutterlauge erhalten.

Ohne die ebenfalls noch 1,1-GPM enthaltenen Mutterlaugen weiter aufzubereiten, konnten mit dem erfindungsgemäßen Verfahren über 20 Gew.-% a.TS des in hydrierter Isomaltulose enthaltenen 1,1-GPM in reiner Form erhalten werden.

### Beispiel 3:

### Herstellung von reinem 1,6-GPS

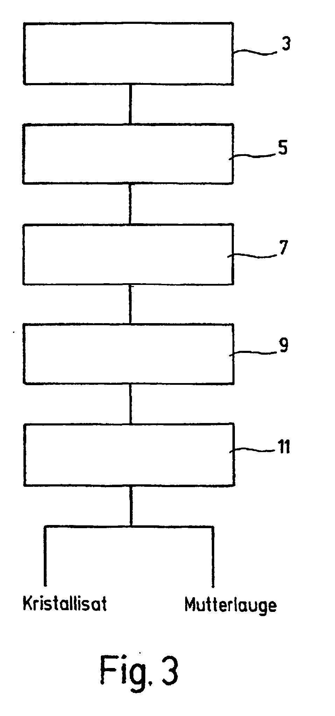

Ausgehend von der in Beispiel 1 erhaltenen 1,6-GPS-reichen Mutterlauge mit einem 1,6-GPS-Anteil von 84 Gew.% a.TS wurden zunächst 560 kg dieser Mutterlauge bei 55 °C aufkonzentriert (Verfahrensschritt 3, Figur 3). Im Gegensatz zur Kristallisation von 1,1-GPM muß hier die schwerer kristallisierbare Komponente 1,6-GPS neben der leicht kristallisierbaren Komponente 1,1-GPM auskristallisiert werden. Dazu ist eserfindungsgemäßbevorzugt, den hohen 1,6-GPS-Anteil in der eingesetzten Mutterlauge zu nutzen, um eine Übersättigung des 1,6-GPS bei geringem Trockensubstanzgehalten der Gesamtlösungen zu erreichen. Bei der Aufkonzentrierung von 1,6-GPS (Verfahrensschritt 3, Figur 3) sollte eine Lösungstemperatur von 55 °C nicht überschritten werden, um ein Schmelzen von 1,6-GPS-Kristallen bei der anschließenden Kühlungskristallisation zu verhindern. Die sich in Verfahrensschritt 5 anschließende Kühlungskristallisation (Verfahrensschritt 5, Figur 3) von 55 °C auf 40 °C sollte eine kleine Kühlrate aufweisen, so daß sich vorwiegend nur 1,6-GPS-Keime bilden können. Dabei wird aufgrund möglichst minimaler Kühltemperatur vermieden, daß eine spontane Kristallisation von 1,1-GPM auftritt. In dem Abkühlbereich von 55 °C bis 40 °C sieht die Erfindung eine Abkühlrate von 0,3 K/h vor. Im Anschluß daran wird eine weitere Aufkonzentrierung bei 55 °C durchgeführt (Verfahrensschritt 7, Figur 3). Anschließend ist eine zweite Kühlungskristallisation von 55 °C auf 40 °C vorgesehen, die in einem Temperaturbereich von 55 °C bis 40 °C eine Abkühlrate von 0,3 K/h vorsieht (Verfahrensschritt 9, Figur 3). Der nach der zweiten Abkühlung entstandene Kristallbrei wird 45 Minuten bei 1.800 U/min zentrifugiert (Verfahrensschritt 11, Figur 3). Das Kristallisat (138 kg) ist 1,6-GPS-reich und weist einen 1,6-GPS-Gehalt von 99 Gew.-% a.TS auf. Die aus der Zentrifugation resultierende Mutterlauge ist ebenfalls 1,6-GPS-reich.

Wie in Beispiel 2 bereits ausgeführt, könnte eine Erhöhung der Produktausbeute erreicht werden, indem anfallende Mutterlaugen weiterverarbeitet werden.

### Beispiel 4:

### Trocknung des reinen 1,1-GPM und reinen 1,6-GPS

Zur Verbesserung der Lagerfähigkeit von 1,1-GPM und 1,6-GPSwerden die in den vorstehenden Beispielen aufgeführten Produkte getrocknet. Die Trocknung wird in einem temperierten diskontinuierlichen Taumeltrockner (Kontakttrockner) unter Vakuum durchgeführt. Der rotierende Behälter des Taumeltrockners sorgt für eine ständige Durchmischung von trocknem und feuchten Gut, wodurch eine gleichmäßige Trocknung erreicht wird. Aufgrund des angelegten Vakuums von < 0,02 bar werden neben der Feuchtigkeit auch feinste Partikel aus dem Trockner abgesaugt. Diese Partikel werden in einem Zyklon wieder abgeschieden.

Zur Trocknung von 1,6-GPS wird dieses 5 Stunden auf 45 °C erhitzt. Dabei wird der Wassergehalt des 1,6-GPS von 2 auf ca. 0,2 Gew.-% a.TS verringert. Eine Klumpenbildung wurde weitgehend vermieden.

Im Gegensatz zu 1,6-GPS enthält 1,1-GPM Kristallwasser. Bei der Trocknung von 1,1-GPM ist daher zusätzlich die Kristallwasserabgabe bei ca. 80 °C zu berücksichtigen. Die Kristallwasserabgabe könnte zu einem Lösen des 1,1-GPM und damit zu einer Verbakkung des getrockneten Gutes führen. Deshalb wird erfindungsgemäß mit einer Heizrate von 1 K/h bei einer Temperatur von 35 °C begonnen. Nach 6 Stunden ist das Produkt trocken und enthält nur noch Kristallwasser. Während der Trocknung verringert sich der Wasseranteil von 9,4 auf 9,3 Gew.-% a.TS. Auch hier wurde eine Klumpenbildung weitgehend vermieden.

## Patentansprüche

1. Verfahren zur Herstellung 1,1-GPM-(1-O-α-D-Glucopyranosyl-D-mannit) und/oder 1,6-GPS-(6-O-α-D-Glucopyranosyl-D-sorbit) angereicherter Phasen, wobei
(i) hydrierte Isomaltulose oder ein hydrierte Isomaltulose-haltiges Gemisch in Wasser bei erhöhter Temperatur gelöst, wobei die erhaltene Lösung eine Konzentration von 70 bis 90 Gew.-% a. TS an hydrierter Isomaltulose oder hydrierte Isomaltulose-haltigem Gemisch aufweist,
(ii) mit der erhaltenen Lösung eine Kühlungskristallisation über eine Temperaturspanne von 90°C bis 37°C durchgeführt wird, wobei die Kühlungskristallisation im Temperaturbereich von 90°C bis 65°C mit einer Kühlrate von 5 bis 15 K/h und in einem Temperaturbereich von 65°C bis 37°C mit einer Kühlrate von 0,4 bis 3 K/h durchgeführt wird, und
(iii) anschließend die 1,1-GPM angereicherte Phase von der 1,6-GPS angereicherten Phase abgetrennt wird.

2. Verfahren nach Anspruch 1, **dadurch gekennzeichnet, daß** in einem Temperaturbereich von 90°C bis 65°C eine Kühlrate von 8 bis 12 K/h eingehalten wird.

3. Verfahren nach einem der Ansprüche 1 oder 2, **dadurch gekennzeichnet, daß** in einem Temperaturbereich von 65°C bis 37°C eine Kühlrate von 0,8 bis 1,5 K/h eingehalten wird.

4. Verfahren nach einem dervorhergehenden Ansprüche, **dadurch gekennzeichnet, daß** hydrierte Isomaltulose oder ein hydrierte Isomaltulose-haltiges Gemisch in Wasser bei einer erhöhten Temperatur von 80°C bis 90°C, insbesondere 85°C, gelöst wird.

5. Verfahren nach einem dervorhergehenden Ansprüche, **dadurch gekennzeichnet, daß** die erhaltene Lösung eine Konzentration an hydrierter Isomaltulose oder hydrierte Isomaltulose-haltigen Gemisch von 75 bis 85 Gew.-% a. TS aufweist.

6. Verfahren nach einem dervorhergehenden Ansprüche, **dadurch gekennzeichnet, daß** während der Kühlungskristallisation eine Animpfung mit hydrierter Isomaltulose, 1,1-GPM oder 1,6-GPS durchgeführt wird.

7. Verfahren nach einem dervorhergehenden Ansprüche, **dadurch gekennzeichnet, daß** die Animpfung mit einer Suspension durchgeführt wird, insbesondere einer Suspension von hydrierter Isomaltulose in Wasser, einem organischen Lösungsmittel, insbesondere Isopropanol, gegebenenfalls unter Verwendung eines lebensmittelverträglichen Dispergierhilfsmittels.

8. Verfahren nach einem dervorhergehenden Ansprüche, **dadurch gekennzeichnet, daß** die Animpfung bei einer Temperatur von 50°C bis 65°C, vorzugsweise 61 °C bis 63°C, durchgeführt wird.

9. Verfahren nach einem der vorhergehenden Ansprüche, **dadurch gekennzeichnet, daß** im Anschluß an die Kühlungskristallisation eine Abtrennung der erhaltenen 1,1-GPM angereicherten Phase von der erhaltenen 1,6-GPS angereicherten Phase mittels Zentrifugation durchgeführt wird.

10. Verfahren zur Herstellung von reinem 1,1-GPM, **dadurch gekennzeichnet, daß** im Anschluß an eines der Verfahren nach einem der vorhergehenden Ansprüche die erhaltene 1,1-GPM angereicherte Phase aufkonzentriert und einmal, gegebenenfalls zwei- oder mehrmals, kühlungskristallisiert wird.

11. Verfahren nach Anspruch 10, **dadurch gekennzeichnet, daß** die 1,1-GPM angereicherte Phase vor der Aufkonzentrierung gereinigt, insbesondere filtriert, wird.

12. Verfahren zur Herstellung von reinem 1,6-GPS, **dadurch gekennzeichnet, daß** im Anschluß an eines der Verfahren nach einem der Ansprüche 1 bis 9 die erhaltene 1,6-GPS-angereicherte Phase mindestens einmal, vorzugsweise zweimal, aufkonzentriert und mindestens einmal, vorzugsweise zweimal, kühlungskristallisiert wird.

13. Verfahren nach einem dervorhergehenden Ansprüche, **dadurch gekennzeichnet, daß** im Anschluß an die Kühlungskristallisation und die gegebenenfalls erfolgende Abtrennung oder Aufkonzentrierung 1,1-GPM oder 1,6-GPS oder das 1,1-GPM- oder das 1,6-GPS-angereicherte Gemisch auf 60 bis 90 % Trockensubstanz eingedickt und mittels einer Verdampfungskristallisation ein trockenes Produkt erhalten wird.

14. Verfahren nach Anspruch 12, **dadurch gekennzeichnet, daß** im Anschluß an die Kühlungskristallisation eine Trocknung des erhaltenen 1,6-GPS fünf Stunden bei 45°C durchgeführt wird.

15. Verfahren nach einem der Ansprüche 10 oder 11, **dadurch gekennzeichnet, daß** im Anschluß an die Kühlungskristallisation eine Trocknung des erhaltenen 1,1-GPM bei einer Temperatur von 35°C beginnt und mit einer Heizrate von 1 K/h für 6 Stunden durchgeführt wird.

16. Verfahren nach einem der vorhergenannten Ansprüche, **dadurch gekennzeichnet, daß** das hydrierte Isomaltulose-haltige Gemisch 1,1-GPM, 1,6-GPS und gegebenenfalls Mannit, Sorbit, 1,1-GPS (1-O-α-D-Glucopyranosyl-D-sorbit), Isomelezitose, Saccharose oder Isomaltose enthält.

## Claims

1. Process for producing 1,1-GPM-(1-O-α-D-glucopyranosyl-D-mannitol) and/or 1,6-GPS-(6-O-α-D-glucopyranosyl-D-sorbitol) enriched phases, wherein
(i) hydrogenated isomaltulose or a mixture containing hydrogenated isomaltulose is dissolved in water at an elevated temperature, wherein the solution obtained has a concentration of hydrogenated isomaltulose or of a mixture containing hydrogenated isomaltulose of 70 to 90% solid phase content by weight,
(ii) cooling crystallisation is carried out with the solution obtained over a temperature range of 90°C to 37°C, wherein cooling crystallisation is carried out within a temperature range of 90°C to 65°C at a cooling rate of 5 to 15 K/h and within a temperature range of 65°C to 37°C at a cooling rate of 0.4 to 3 K/h; and
(iii) subsequently the 1,1-GPM-enriched phase is separated from the 1,6-GPS-enriched phase.

2. Process according to claim 1, **characterised in that** a cooling rate of 8 to 12 K/h is adhered to within a temperature range of 90°C to 65°C.

3. Process according to either of claims 1 or 2, **characterised in that** a cooling rate of 0.8 to 1.5 K/h is adhered to within a temperature range of 65°C to 37°C.

4. Process according to any of the preceding claims, **characterised in that** hydrogenated isomaltulose or a mixture containing hydrogenated isomaltulose is dissolved in water at an elevated temperature of 80°C to 90°C, in particular 85°C.

5. Process according to any of the preceding claims, **characterised in that** the solution obtained has a concentration of hydrogenated isomaltulose or of a mixture containing hydrogenated isomaltulose of 75 to 85% solid phase content by weight.

6. Process according to any of the preceding claims, **characterised in that** seeding with hydrogenated isomaltulose, 1,1-GPM or 1,6-GPS is carried out during cooling crystallisation.

7. Process according to any of the preceding claims, **characterised in that** the seeding is carried out using a suspension, in particular a suspension of hydrogenated isomaltulose in water, an organic solvent, in particular isopropanol, optionally while using a food-compatible dispersing agent.

8. Process according to any of the preceding claims, **characterised in that** the seeding is carried out a temperature of 50°C to 65°C, preferably 61°C to 63°C.

9. Process according to any of the preceding claims, **characterised in that** after cooling crystallisation, the 1,1-GPM-enriched phase obtained is separated from the 1,6-GPS-enriched phase obtained by means of centrifugation.

10. Process for producing pure 1,1-GPM, **characterised in that** after a process according to any of the preceding claims, the 1,1-GPM-enriched phase obtained is concentrated and cooling crystallised once, optionally twice or several times.

11. Process according to claim 10, **characterised in that** the 1,1-GPM-enriched phase is purified, in particular filtered, before concentration.

12. Process for producing pure 1,6-GPS, **characterised in that** after a process according to any of claims 1 to 9, the 1,6-GPS-enriched phase obtained is concentrated at least once, preferably twice and cooling crystallised at least once, preferably twice.

13. Process according to any of the preceding claims, **characterised in that** after cooling crystallisation and the optional separation or concentration, 1,1-GPM or 1,6-GPS or the 1,1-GPM-enriched or the 1,6-GPS-enriched mixture is thickened to 60 to 90% dry substance and a dry product is obtained by means of evaporative crystallisation.

14. Process according to claim 12, **characterised in that** after cooling crystallisation, the 1,6-GPS obtained is dried for 5 hours at 45°C.

15. Process according to claim 10 or 11, **characterised in that** after cooling crystallisation, the 1,1-GPM obtained is dried for 6 hours at an initial temperature of 35°C using a heating rate of 1 K/h.

16. Process according to any of the preceding claims, **characterised in that** the mixture containing hydrogenated isomaltulose contains 1,1-GPM, 1,6-GPS, and optionally mannitol, sorbitol, 1,1-GPS (1-0-α-D-glucopyranosyl-D-sorbitol), isomelezitose, saccharose, or isomaltose.

## Revendications

1. Procédé de préparation de phases enrichies en 1,1-GPM-(1-O-α-D-glucopyranosyl-D-mannitol) et/ou en 1,6-GPS-(6-O-α -D-glucopyranosyl-D-sorbitol), dans lequel
(i) on dissout dans l'eau à une température élevée de l'isomaltulose hydrogéné ou un mélange contenant de l'isomaltulose hydrogéné, dans lequel la solution obtenue présente une concentration en isomaltulose hydrogéné ou en mélange contenant de l'isomaltulose hydrogéné de 70 à 90% en poids en M.S. (matière sèche) ;
(ii) on effectue, sur la solution obtenue, une cristallisation par refroidissement en passant d'une température de 90°C à 37°C, la cristallisation par refroidissement s'effectuant dans l'intervalle de températures de 90°C à 65°C à une vitesse de refroidissement de 5 à 15 K/h et dans l'intervalle de températures de 65°C à 37°C à une vitesse de refroidissement de 0,4 à 3 K/h, et
(iii) on sépare ensuite la phase enrichie en 1,1-GPM de la phase enrichie en 1,6-GPS.

2. Procédé selon la revendication 1, **caractérisé en ce qu'**on maintient, dans un intervalle de températures de 90°C à 65°C, une vitesse de refroidissement de 8 à 12 K/h.

3. Procédé selon l'une des revendications 1 ou 2, **caractérisé en ce qu'**on maintient, dans un intervalle de températures de 65°C à 37°C, une vitesse de refroidissement de 0,8 à 1,5 K/h.

4. Procédé selon l'une des revendications précédentes, **caractérisé en ce qu'**on dissout dans l'eau à une température élevée de 80°C à 90°C, en particulier de 85°C, l'isomaltulose hydrogéné ou un mélange contenant de l'isomaltulose hydrogéné.

5. Procédé selon l'une quelconque des revendications précédentes, **caractérisé en ce que** la solution obtenue présente une concentration en isomaltulose hydrogéné ou en mélange contenant de l'isomaltulose hydrogéné de 75 à 85% en poids en M.S (matière sèche).

6. Procédé selon l'une quelconque des revendications précédentes, **caractérisé en ce qu'**on effectue, pendant la cristallisation par refroidissement, une inoculation avec de l'isomaltulose hydrogéné, du 1,1-GPM ou du 1,6-GPS.

7. Procédé selon l'une quelconque des revendications précédentes, **caractérisé en ce que** l'inoculation s'effectue avec une suspension, en particulier avec une suspension d'isomaltulose hydrogéné dans l'eau, un solvant organique, en particulier de l'isopropanol, le cas échéant en utilisant un agent de dispersion compatible avec les substances alimentaires.

8. Procédé selon l'une quelconque des revendications précédentes, **caractérisé en ce que** l'inoculation s'effectue à une température de 50°C à 65°C, de préférence de 61°C à 63°C.

9. Procédé selon l'une quelconque des revendications précédentes, **caractérisé en ce qu'**on effectue par centrifugation, à la fin de la cristallisation par refroidissement, une séparation de la phase enrichie en 1,1-GPM obtenue de la phase enrichie en 1,6-GPS obtenue.

10. Procédé de préparation de 1,1-GPM pur, **caractérisé en ce qu'**à la fin d'un des procédés selon l'une quelconque des revendications précédentes, la phase enrichie en 1,1-GPM obtenue est concentrée et se cristallise par refroidissement une fois, le cas échéant deux fois ou plus.

11. Procédé selon la revendication 10, **caractérisé en ce que** la phase enrichie en 1,1-GPM est purifiée, en particulier, filtrée avant la concentration.

12. Procédé de préparation de 1,6-GPS pur, **caractérisé en ce que**, à la fin d'un des procédés selon l'une quelconque des revendications 1 à 9, la phase enrichie en 1,6-GPS obtenue est concentrée au moins une fois, de préférence deux fois et est cristallisée par refroidissement au moins une fois, de préférence deux fois.

13. Procédé selon l'une quelconque des revendications précédentes, **caractérisé en ce que**, à la fin de la cristallisation par refroidissement et la séparation ou concentration ultérieure éventuelle, le 1,1-GPM ou le 1,6-GPS ou le mélange enrichi en 1,1-GPM ou en 1,6-GPS est épaissi à 60 à 90% de matière sèche, et on obtient, par cristallisation par évaporation, un produit sec.

14. Procédé selon la revendication 12, **caractérisé en ce que**, à la fin de la cristallisation par refroidissement, on effectue un séchage du 1,6-GPS obtenu pendant cinq heures à 45°C.

15. Procédé selon l'une des revendications 10 ou 11, **caractérisé en ce que**, à la fin de la cristallisation par refroidissement, commence un séchage du 1,1-GPM obtenu à une température de 35°C, qui s'effectue à une vitesse de chauffage de 1 K/h pendant 6 heures.

16. Procédé selon l'une quelconque des revendications précédentes, **caractérisé en ce que** le mélange contenant de l'isomaltulose hydrogéné contient du 1,1-GPM, du 1,6-GPS et le cas échéant du mannitol, du sorbitol, du 1,1-GPS (1-O-α-D-glucopyranosyl-D-sorbitol), de l'isomélécitose, du saccharose ou de l'isomaltulose.
